Europäisches Patentamt

European Patent Office

Office européen des brevets

(11) Publication number: **0 131 878**
**A2**

# (12) EUROPEAN PATENT APPLICATION

(21) Application number: **84107961.9**

(22) Date of filing: **19.07.84**

(51) Int. Cl.⁴: **C 12 N 15/00**
C 12 N 5/00, C 12 P 21/00
//G01N33/577, A61K39/42

(30) Priority: **19.07.83 US 515288**

(43) Date of publication of application:
**23.01.85 Bulletin 85/4**

(84) Designated Contracting States:
**DE FR GB**

(71) Applicant: **Sloan-Kettering Institute For Cancer Research**
**1275 York Avenue**
**New York New York 10021(US)**

(72) Inventor: **Hammerling, Ulrich**
**430 East 63 Street**
**New York New York 10021(US)**

(72) Inventor: **Hoffman, Michael K.**
**132 Greene Street**
**New York New York 10012(US)**

(74) Representative: **Patentanwälte Schulze Horn und Hoffmeister**
**Goldstrasse 36**
**D-4400 Münster(DE)**

(54) Process for producing human monoclonal antibodies.

(57) A method for producing human monoclonal antibodies from human B-lymphocytes comprising the steps:

1. immunization of human B-lymphocytes;

2. transformation of HBL by a viral agent to generate immortalized cells;

3. selection of cells secreting specific antibodies and cloning of these cells; and

4. fusion of the cloned cells with suitable human or mouse myeloma cells to generate high-secreting hybridoma, and cloning of these hybridomas is disclosed.

The method may be used to produce human monoclonal antibodies which recognize HLA, A, B, C or DR antigens and also to produce human monoclonal antibodies recognizing cytomegalo-virus antigens. Human monoclonal antibodies deposited at the American Type Culture Collection with deposit numbers HB 8317 and CRL 8316 have been produced by this method.

# Process For Producing Human Monoclonal Antibodies

This invention concerns human monoclonal antibodies. A novel method for their production involving immortalization of human antibody-secreting cells prior to fusion and conversion to hybridoma cells is disclosed. Human monoclonal antibodies recognizing human cell surface antigens and viruses associated with the herpes group are disclosed.

## Summary

The present invention is concerned with a method for producing human monoclonal antibodies from human B-lymphocytes which comprises the following:

1. immunization of human B-lymphocytes (HBL) in tissue culture,

2. transformation of HBL by a viral agent, preferrably Epstein-Barr virus to generate immortalized cells;

3. selection of cells secreting specific antibodies and cloning of these cells; and

4. fusion of the cloned cells with suitable human or mouse myeloma cells to generate high-secreting hybridomas, and cloning of these hybridomas.

The method may be used, for example, to prepare human monoclonal antibodies which recognize HLA, A, B, C or DR antigen by transforming PBL which have been immunized in vitro with the antigen or by transforming PBL from blood of multiparous women with high anti-HLA and/or DR antibody titers.

The method may also be used to produce human monoclonal antibodies recognizing cytomegalo virus antigens by transforming PBL from an individual exposed to cytomegalo virus with a viral agent, preferrably Epstein-Barr virus to generate immortalized cells and fusing the transformed cells with a second immortal cell line. Human monoclonal antibodies designated 312 A 91.4 and 311/25 have been produced by this method.

Background

Successful production of murine B-lymphocyte hybridomas made it possible to manufacture antibodies with restricted specificities in large quantities. However, attempts to produce human monoclonal antibodies with the methodologies established for the murine antibodies have failed.

Human monoclonal antibodies to human cell surface antigens, especially the major histocompatibility complex (MHC) have been sought. A number of mouse monoclonal antibodies (mAb) to HLA, A, B, C surface antigens and to DR antigens have been prepared (Brodsky, F. M., Parham, P., Barnstable, C. J., Crumption, M. J., and Bodmer, W. F. Immunol. Rev. 47:3 1979; Trucco, M., Garotta, J. W., Strocker, J. W., and Cepellini, R. Immunol. Rev. 47:219, 1979; Trucco, M., Stocker, J. W. and Cepellini, R.

Nature 273:666, 1978; Quaranta, V., Pellegrino, M. A. and Ferrone S. In: Monoclonal antibodies and T cell Hybridomas. Eds. G. J. Hammerling, U. Hammerling and J. F. Kearney. Elsevier North Holland, Amsterdam, p. 84, 1981; Lampson, L. A. and Levy, R. J. Immunol. 125:293, 1980). These antibodies have been showed to react with antigenic sites common to all MHC antigens, but except in isolated instances do not react with polymorphic determinants of the human MHC. General experience in immunogenetics has taught the value of alloimmunizations and the fine specificities of the resulting alloantibodies. Accordingly, only alloantibodies are regarded as suitable to chart the genetic and serological complexities of the human MHC, and hence ultimately provide the reagents necessary for human tissue typing. It is to be expected that relevant monoclonal antibodies will derive sooner from alloimmune than from heteroimmune lymphocytes. Previous human monoclonal antibodies to human cell surface antigens, notably to MHC antigens are not known.

Human monoclonal antibodies associated with viruses of the herpes group have also been sought. The herpes virus group consists of a large number of viruses which are either etiological agents or found associated with numerous human illnesses. They include Herpes type 1 and type 2, Cytomegalovirus, Varicella Zoter, Epstein-Barr Virus, among others. Cytomegalovirus (CMV) is in itself a complex group of viruses, the members of which can cause many diseases in man. Serological classifications of CMV in pertinent serotypes is incomplete at best. Monoclonal antibodies would provide ideal reagents for serotyping. Human monoclonal antibodies would also be important adjuncts for the treatment of CMV infections, as implied from the beneficial use of immune gamma globulin in patients. To date, human monoclonal antibodies with specificity to CMV have not been produced.

It is now clear that the biology of human B-lymphocytes necessitates a total change of approach from that used in the murine methodology in order to produce human monoclonal antibodies.

## Description

The present invention will be more fully understood from the following description taken in conjunction with the accompanying drawing wherein Figure 1 depicts the virus transformation step of the method.

Human monoclonal antibodies obtained by the method disclosed in the present invention bear the designated deposit number and are deposited with Sloan-Kettering Institute, 1275 York Avenue, New York, New York 10021. Preferred cell lines of the present invention are also deposited at the American Type Culture Collection, Bethesda, Maryland and bear the following deposit numbers:

| SK Deposit Number | ATCC # |
|---|---|
| 312 A 91.4 | HB 8317 |
| 311/125 | CRL 8316 |

Deposit is for the purpose of enabling disclosure only and is not intended to limit the concept of the present invention to the particular materials deposited.

The terminology "lymphcyte" and "cell" is used interchangeably in this application - as is usual in the art.

In accordance with the inventive method, a reliable source of immunized human B-lymphocyte must be obtained. Preferrably normal peripheral blood lymphocytes constitute that source. Towards this goal a culture method has

been developed (Hoffman, M. K. Proc. Nat'l. Acad. Sci. USA 77, 1139, 1980; Lane, H. C. et al. J. Exp. Med. 154, 1043, 1981; Misiti, J. et al J. Exp. Med. 154, 1069, 1981; Morimoto, C. et al. J. Immunol. 127, 514, 1981) that permits effective immunization of unsensitized human PBM to sheep erythrocytes (SRBC) or antigens conjugated to SRBC (such as trinitrophenyl groups, TNP) and immunization of presensitized human lymphocytes to other antigens (tetanus toxoid, alloantigens). Specific B-lymphocytes obtained in this method can be readily transformed according to the present invention with EB virus and TNP specific as well as other antigen specific transformed B cell lines have thus been obtained. For convenience, the PBL, once obtained, may be cryopreserved prior to immunization.

It is possible that antibody-secreting human B cells, as terminally differentiated cells, have in their biological life cycle a limited allowance of generation cycles. It is conceivable such a block is a dominant feature which might negatively influence the fusion cell partner. The contention that human plasma cells have a limited potential for proliferation is supported by the low frequency of human plasmacytomas, the general difficulty of adapting such plasmacytomas to cell culture, the reported failure to clone immunoglobulin secreting plasma cell type progeny of EBV transformed B cells (Fu, S. M., et al J. Exp. Med. 148, 1570, 1978), and the inability to maintain normal human B cells in the PFC in culture. In fact, the situation may not be different in principal from that of mouse plasmacytomas which are infrequent in most mouse strains except the BALB/c strain (Potter, M. Methods in Cancer Research. Ed. H. Busch. Academic Press. Vol. 2, 1967, p. 105).

Based on this hypothesis the inventive method was devised to circumvent the possibility of a biological block using an adaptation of EBV transformation technique as a means of immortalizing and cloning human allo-antibody-forming cells to human lymphocyte surface antigens, specifically to HLA-A, B, C and DR antigens. Clones of low-secreting cells may be then turned into high-secreting cells by fusion with standard murine plasmacytoma cells or human myeloma cells.

The inventive method for generating human monoclonal antibodies (mAb) comprises essentially the steps:

(1) human B lymphocytes are sensitized or immunized using a suitable antigen; immunization is preferrably $\underline{in\ vitro}$ but sensitized PBL of high titer to a specific antigen may also be employed;

(2) the antibody producing cell lines are then immortalized by EB virus transformation;

(3) the transformed cells are fused with a second immortal cell lines, a mouse myeloma cell line, for example.

The transformed cell lines are then cloned -- that is, after culturing, one selects those cell colonies which have the desired characteristics --; and the cloned cell lines are grown to produce the desired antibodies. The method may comprise, in addition, the steps of cloning transformed cells, screening the cloned transformed cells for antibody secretion and selecting for fusion the efficient antibody secretors - that is, those which secrete the larger amounts of antibody under the cloning conditions.

The following examples are provided to illustrate the method of the present invention and the human monoclonal antibodies that have been obtained thereby. They are in no way, however, intended to limit the scope of the invention.

EXAMPLES

I. Human mAb to nominal antigens, sheep erythrocytes (SRBC) and trinitrophenyl groups (TNP)

a) Isolation of human peripheral blood lymphocytes (PBL): This procedure follows standard ficoll-hypague density gradient centrifugation. 50 milliliters of human blood are diluted with a twofold volume of Earles balanced salt solution (EBSS), layered on lymphoprep (Ryegard, Denmark) and centrifuged at room temperature for 30 min. at 1800 RPM. The cells from the interface are isolated, washed in EBSS and counted.

b) In vitro immunization of PBL with TNP-SRBC. $40 \times 10^6$ Human PBL suspended 10 milliliters of in Mishell Dutton medium (Mishell, R. I. and Dutton, R. W. J. Exp. Med. 126:423) containing 5 % fetal calf serum (FBS), antibiotics, $5 \times 10^{-5}$ 2-mercaptoethanol (2-ME). Thirty microliters of a 50 % suspension of heat-killed staphylococcus aureus, strain Cowan I, 300 microliters of B cell differentiation factors (BDF) (Hoffmann, M. K., Proc. Nat'l. Acad. Sci. USA 77:1139, 1980), and 50 microliters of a 6 % TNP-SRBC suspension are added to this cell suspension, and the mixture is then distributed evenly into one 96-well Costar plate. The cells are cultured for 18 hours in humidified $CO_2$ incubators, and 10 microliters of human AB serum is added to each well. Culturing is continued for 4 days with intermittant feeding by 10 microliters of feeding cocktail (Mishell, et al.

Supra) every other day. The cells are then harvested, washed in EBSS and counted. Antibody formation is assessed by the Jerne plaque assay (Jerne, N. K. and Nordin, A. A. Science 140:405, 1963) using TNP-horse erythrocytes and SRBC.

c) Isolation of B cells and transformation by Epstein-Barr virus (EBV)

Immunized PBL are adjusted to $4 \times 10^6$ cells per ml in medium containing 5 % FBS. 10 milliliters of the mixture is added to petridishes coated with polyvalent affinity-purified rabbit anti-human Ig antibody. The dishes are prepared by incubating 10 cm microbiological-grade plastic dishes with 10 ml of the antibody at 10 micrograms/ml overneight in the refrigerator, followed by blocking residual sites by incubation with a 10 % solution FBS in EBSS for 1 hr. at $37^o$ C. B cells are allowed to attach during 3 hrs. at $4^o$ C. The plates are then washed gently with EBSS twice to remove T cells. 10 ml of a virus solution containing high-titers of EBV are added to the plate which is then transferred to a $CO_2$-incubator overnight. The virus suspension used is the culture fluid of the marmoset cell line B95-8 as described (Miller, G. and Lipman, M. Proc. Nat'l. Acad. Sc. U. S. A. 70:190, 1973). After overnight culture the B cells are harvested from the petridish, counted and transferred to 96-well plates containing a confluent layer of human fibroblasts (e. g. Flow 5000 cells). $40 \times 10^4$ cells are seeded in each well. Cultures are fed twice weekly. The culture medium is RPMI 1640 containing 10 % FBS, antibiotics and 2-ME.

This EBV-transformation scheme is depicted in Figure 1.

d) Screening of culture supernates for antibody.

Supernates are harvested when the cultures have grown to 1 to 2 x $10^5$ cells per well. The antibody content is measured by hemagglutiuation using TNP-SRBC, SRBC alone, and TNP-horse RBC. In parallel, ELISA assays are set up with TNP-conjugated to bovine serum albumin (G. J. Hammerlin, and J. Kearney. Monoclonal antibodies and T cell hybridomas. Perspectives and technical advances. Appendix, Elsevier North Holland, 1981, p. 574). The developing antibody is polyvalent, affinity purified anti human IgG coupled with alkaline phosphatase.

Antibody-positive cultures so identified are propagated further. Part of the cells are frozen for future reference.

e) Cloning of EBV transformed cells. Precloning Original cultures of EBV transformed cells contain many isolated clusters of cells. As cells within each cluster are relatively homogeneous individual clusters are hand picked with the aid of a capillary pipet and transferred to new 96-well plates containing confluent monolayers of irradiated human fibroblasts. Cells are allowed to grow to 1 x $10^5$/well and supernates are again tested for antibody by a suitable method, the method described hereinabove at 1d, for example. Antibody-positive cultures are again propagated by transfer of individual clusters, or stored in liquid nitrogen for future use.

Cloning by growth in soft agar. This procedure is well known in the art, as for example disclosed by Sugden, B. and Mark, W. J. in Virol. 23:503 (1977). Experimental results are given in Tables 1 and 2.

## Table 1

| Exp.No. | No.of cultures | No.of antibody positive wells | | No.of cell lines stable after 2 mos. | No.cell lines with 20 % rosette-for-ming cells |
|---------|----------------|---------|---------|----------------|-----------------|
| | | TNP | SRBC | | |
| 216 | 192 | 15 | 4 | 14 | 5 |
| 223 | 500 | 22 | 13 | 10 | 2 |
| 234 | 360 | 10 | 6 | n. a. | n. a. |

## Table 2

| Clone # | No.of cells seeded | No.of colonies growing | No.of. clones picked | No. of antibody positive clones | Ig class |
|---------|--------------------|-----------------|---------------|-------------------|----------|
| 223/38 | $10^4$ | 500 | 24 | 2 | |
| 223/97 | $10^4$ | 300 | 24 | 3 | |
| 223/187 | $10^4$ | 600 | 24 | 1 | * |
| 223/115 | $10^4$ | 800 | 24 | 0 | |
| 223/168 | $10^4$ | 500 | 24 | 0 | – |
| 223/287 | $10^4$ | none | -- | -- | – |

*recloning required

Legend to Tables 1 and 2:

100 ml of fresh heparinized blood was fractionated on ficoll-hypaque gradients, yielding 1.0 to 1.25 x $10^8$ PBM. These cells were stimulated in culture with TNP-SRBC as described under "technical notes". Five to seven days later the B cells were isolated by passing the cells over plastic dishes coated with affinity-purified polyvalent rabbit anti-human IgG, using one 10 cm dish per 4 x $10^7$ cells. From 1.2 to 2.5 x $10^7$ B cells were recovered as the adherent fraction. They were 90 to 95 % Ig+ by immunofluorescence. B cells were infected with 3 ml of undiluted culture fluid of the marmoset cell line B958. Cells were than diluted with RPMI medium containing 10 % FBS to 2 x $10^5$ cells/ml and seeded on 96-well plates. These contained a confluent layer of human fibroblasts and were irradiated with 4000 rads before use. Each well received 2 x $10^4$ B cells. Growth was noticeable after 2 weeks, and after 4 weeks cultures were tested for antibody using three assays:

1) addition of TNP-SRBC to wells and reading hemagglutination patterns,

2) hemagglutination to anti-TNP-SRBC, TNP horse and SRBC in Terasaki plates,

3) ELISA with TNP-BSA as antigen.

Twenty-three cultures showed antibody activity to SRBC and 47 to TNP (see Table 2). Cells from positive cultures were frozen in liquid nitrogen. Of the 35 cultures carried further, 24 discontinued making antibody; some, probably due to overgrowth by irrelevant cells, at a declining rate. To estimate the number of TNP-specific cells, antibody rosettes were allowed to form with TNP-SRBC. Of 20 cultures so tested, seven produced 10 % or more rosettes, five had from 1 to 10 %, and the rest were either negative, showed occasional rosettes, or were ambiguous (clumps due to dead cells).

Attempts to clone anti-TNP secreting cells in agarose were in part successful. Ten-thousand cells of 6 selected anti-TNP cultures were grown in 0.35 % agarose, on top of an irradiated fibroblast monolayer overlayed with 0.5 % agarose, using RPMI medium. Colonies developed in five of the six cultures within 3 weeks. They were picked under the stereomicroscope, transferred to irradiated fibroblast feeders and grown to $10^4 - 10^5$ cells/well. When tested by ELISA, 3 of 5 anti-TNP cell lines yielded positive clones with varying frequencies (see Table 3). Culture fluids were assayed by the ELISA procedure of J. Kearney (G. J. Hammerling, U. Hammerling, and J. Kearney. Monoclonal antibodies and T cell hybridomas. Perspectives and technical advances. Appendix, Elsevier North Holland, 1981, p. 574). Immunochemical assays to show homogeneity and monoclonality of human Ig are underway. Assays with class-specific and L chain-specific mAb by ELISA showed clones 223/38 and 223/97 to be IgG, whereas clone 223/187 was ambiguous and probably still represents a mixture. These results reinforce our opinion that in vitro immunization of non-immune lymphocytes, rescue of immune cells so generated by EBV transformation, and cloning in soft agar is a viable method for the production of human mAb.

## Cloning by limiting dilution in microcytotoxicity plates Microcytotoxicity plates (Falcon #3034) were seeded with human fibroblasts and grown to confluency. They were irradiated with 2000 rads. Two x $10^3$ normal human B cells prepared by panning (see 1c) were added to each well, followed by the EBV-transformed cells to be cloned, at a rate of one to 5 cells per well. Plates were cultured in a humidified atmosphere in a tissue culture incubator. After 24 hr 10 microliters of medium were added. No further feeding was required. When grown to $10^3$ cells/well EBV transformed cells were transferred to 96-well plates with human fibroblast feeder cells. Selection for antibody was performed as described hereinabove, Section 1d.

Test for clonality:

Two criteria were used:

1) restriction to a single type of L chain and to single class of immunoglobulin. The analysis for light chain expression was done by ELISA assay. Class and L chain specific murine monoclonal antibodies to human Ig were used.

2) Recloning yielded antibody-forming cultured in about 98 % of culture wells.

Cloning in 96-well plates.

An alternate method employed 96-well plates with human irradiated fibroblast as feeder cells. Additional feeder cells were irradiated (1500 rad). Cells of the HPRT-negative variant GM 1500-6TG (Croce, C. M., Linnenbach, A., Hall, W., Steplewski, Z. and Koprowski, H. Nature 288:488, 1980), added to the wells at $10^4$ cells/well. One to five EBV-transformed cells to be cloned were added. Cultures were fed with medium twice a week. After 2 to 3 weeks the culture medium was mixed with 2 % hypoxanthine, aminopterin and thymidine (HAT) to eliminate any growth of feeder cells. Selection for antibody positive cultures is carried out after 4 to 6 weeks, as described hereinabove in Section 1d.

II. Human mAb to lymphocyte surface antigens HLA; DR

a) Immunization in culture Human PBL were obtained from volunteer donors previously identified to have high titers of anti-HLA antibody as a result of pregnancy or blood transfusion. PBL were isolated as described hereinabove under la. PBL were suspended in Mishell-Dutton medium. Irradiated (1500 rad) peripheral blood lymphocytes from the immunized donors (i. e. in case of a pregnancy PBL of the husband or the child) served as antigen. Alternatively an irradiated permanent B cell line derived by EBV transformation from the immunizing donor may be used. Antigen was added at a rate of 0.1 % of PBL to be immunized. The Mishell-Dutton culture was performed as described hereinabove under Ib.

b) Isolation of B cells and transformation This was performed as described hereinabove under Ic.

c) Screening of supernates for antibody to cell surface antigens:

The microcytotoxicity assay was applied. To 5 microliters of supernate were added 1 microliter of a suspension of test cells (usually $2 \times 10^6$ cells/ml of transformed B cells of the immunizing donor) and 2 microliters of selected rabbit complement at 1 : 3 dilution. The assay plates were incubated at $37^{\circ}$ for 45 min., and scored on an inverted microscope after addition of 5 microliters of trypan blue solution.

d) Cloning of transformed cells.

This procedure may be performed as described by one of the methods of Ie.

f) Assay for specificity.

Antibodies were tested by the microcytotoxicity assay on a wide panel of cells according to standard tissue typing protocols. An example is given in Table 3.

Table 3

Evaluation of Antibody Specificaties –
Tests with Frozen Panel Cells

| Supernatant Number | Immunizing Cell Type | Panel Test Results* | |
|---|---|---|---|
| | | T-cells | B-CLL |
| 1537-258/44 AP | A1,2;B8,27;DR1,3 | 18/25 | 2/23 |
| 1540-253/26 | " | 11/25 | 3/23 |
| 1511-258/59 | A2,w31;Bw35,w50 | 21/25 | 17/23 |
| 1515-258/53.10 | " | 0/25 | 7/23 |
| 1535-258/14.20 | " | 4/25 | 0/23 |
| 1536-258/117 | " | 24/25 | 15/23 |
| 1538-258/G43 | " | 13/25 | 1/23 |
| 1539-256/12AP | " | 0/25 | 0/23 |
| 1541-258/G61 | " | 3/25 | 0/23 |
| 1542-258/18.9 | " | 0/25 | 0/23 |
| 1554-266/82.4 | " | 11/25 | 9/23 |

*None of the reaction patterns showed correlations with
known HLA-A, B, C or DR allospecificities.

Antibodies to several distinct alloantigens of
human lymphocytes were obtained. The reactivity
patterns were not congruent with any known HLA
antigens.

III. <u>Human monoclonal antibodies to Cytomegalovirus</u>
<u>(CVM)</u>

a) <u>Immunization in culture</u>

This step is not required, as hyperimmune cells
can be recovered from patients reconvalescent
from cytomegaloinfection, or from chronic CMV-
infected individuals.

b) <u>Transformation</u>

Hence, the PBL obtained from 50 ml of blood were
directly fractionated to yield B cells and these
were transformed with B95-8 EBV als described
under Ib.

c) <u>Screening for antibody to CMV</u>

The ELISA assay is used as described (G. J.
Hammerling, U. Hammerling, and J. Kearney.
<u>Monoclonal antibodies and T cell hybridomas.</u>
<u>Perspectives and technical advances.</u> Appendix,
Elsevier North Holland, 1981, p. 574). 96-well
plates were coated with antigen (CMV CF antigen
Strain AD169 from Flow Laboratories or control
antigen) cell lysate of HEF cells. After blocking
with BSA, culture fluids are transferred to the
plates, which are incubated for 2 hours at room
temperature. Plates were washed with Tween/PBS,
and developing antibody, i. e. rabbit-antihuman
IgG conjugated to alkaline phosphatase, was added. After 2 hours, the plates were washed again
and nitrophenylphosphate substrate was added. The
result was recorded after 1 to 2 hours by an
automatic Elisa-scanner.

d) <u>Cloning by the limiting dilution technique</u>

Clones of cell line 311/125 produce an antibody of IgG, Kappa type.

e) <u>Fusion of EBV-transformed cells with human myeloma cells</u>

$2 \times 10^6$ EBV-transformed cells (not necessarily fully cloned) were mixed with $4 \times 10^6$ human myeloma cells (e. g. the HPRT-negative variant and ouabain-resistant variant of cell lines GM1500. 6TG (Croce, C. M., Linnenbach, A., Hall, W., Steplewski, Z. and Koprowski, H. Nature 288:488, 1980), WIL-2, ARH (American Type Culture Collection). Murine plasmacytoma cell lines X63.5.6.3 can also be used. The cell mixture was centrifuged and the cell pellet mixed with 1 ml PEG 4000 (35 % solution) for 1 min. at RT. The mixture was then diluted dropwise with EBSS at a rate of 15 microliters in the course of 3 minutes. The cells were spun down, taken up in PRMI medium containing 2 % HAT and plated on irradiated human fibroblast feeder cells in 96-well plates at a density of $10^5$ cell per well. Cultures were fed on the second day with $5 \times 10^{-8}$ M ouabain and HAT, and this feeding procedure was continued twice a week thereafter. After 2 weeks cells were harvested, layered on ficoll-hypaque and centrifuged. Viable cells from the interface were plated again as before. When vigorous growth occur supernates were screened for antibody as described in 3c.

f) Characterization of (312a91) anti-CMV antibodies Specificity analysis by ELISA technique with standard CMB antigen AD169 (Flow Laboratories) suggested the presence of anti-CMV antibody, hereinafter designated 312a91.4. Immunoflourescence assays with human fibroblasts infected with AD169 cytomegalo-virus, but not with uninfected cells, corroborates the specificity for CMV, and moreover suggests a relationship to nuclear CMV antigen. The antibody 312a91.4 does not neutralize CMV.

Immunochemical analysis shows that the hybridoma 312a91.4 secretes immunoglobulin G with kappa chain. The anti-CMV antibody binds to Protein A.

What is Claimed:

1.  Method for preparation of hybridomas which produce human monoclonal antibodies recognizing a desired antigen comprising:
    a)  immunizing human peripheral blood lymphocytes (PBL) with said antigen;
    b)  transforming the immunized lymphocytes with a viral agent to generate immortalized cells; and
    c)  fusing the transformed cells with a second immortal cell line.

2.  Method of Claim 1 comprising in addition the step of cloning transformed cells, screening the cloned transformed cells for antibody secretion and selecting the efficient antibody emitters for fusion.

3.  Method of Claim 1 wherein said PBL are immunized in vitro.

4.  Method of Claim 1 wherein said PBL are obtained from individuals exposed to said antigen.

5.  Method of Claim 3 for preparing hybridomas which produce human monoclonal antibodies recognizing HLA, A, B, C antigen or DR antigen wherein human peripheral blood lymphocytes are immunized with said antigen in vitro.

6.  Method of Claim 4 for preparing hybridomas which produce human monoclonal antibodies recognizing HLA-A, B, C or DR antigen wherein said PBL are obtained from the blood of multiparous women with high anti-HLA and/or DR antibody titer.

7.  Method of Claim 1 wherein said viral agent is Epstein-Barr virus.

8. Method of Claim 1 wherein said immortal cell line is of murine or human origin.

9. Method of Claim 8 wherein said immortal cell line is mouse myeloma or a human plasmacytoma.

10. Method of Claim 1 wherein said PBL are cryopreserved prior to immunization.

11. Hybridomas prepared by the method of Claim 1.

12. Human monoclonal antibodies produced by the hybridomas of Claim 11.

13. Hybridomas prepared by the method of Claim 2.

14. Human monoclonal antibodies produced by the hybridomas of Claim 13.

15. Method of Claim 1 for preparing hybridomas which produce human monoclonal antibodies recognizing cytomegalo virus antigens comprising transforming peripheral blood lymphocytes from an individual exposed to cytomegalo virus with a viral agent to generate immortalized cells and fusing the transformed cells with a second immortal cell line.

16. Hybridomas prepared by the method of Claim 15.

17. Human monoclonal antibodies produced by the hybridomas of Claim 16.

18. Human monoclonal antibodies of Claim 17 produced by cell lines deposited at the American Type Culture Collection under the deposit numbers HB 8317 and CRL 8316.